# NEW EUROPEAN PATENT SPECIFICATION

(11) **EP 2 906 547 B2**
(45) Date of publication and mention of the opposition decision: **26.10.2022**
(45) Mention of the grant of the patent: 20.11.2019
(21) Application number: 13844959.0
(22) Date of filing: 11.10.2013
(51) Int. Cl.: C07D 311/00, A61K 8/34, A61K 8/368, A61K 8/49, A61Q 19/00, A61Q 19/08

(54) **COSMETIC COMPOSITIONS CONTAINING AT LEAST ONE HYDROTROPE AND AT LEAST ONE ACTIVE COMPOUND**
KOSMETISCHE ZUSAMMENSETZUNGEN MIT MINDESTENS EINEM HYDROTROP UND MINDESTENS EINEM WIRKSTOFF
COMPOSITIONS COSMÉTIQUES COMPRENANT AU MOINS UN HYDROTROPE ET AU MOINS UN COMPOSÉ ACTIF

(30) Priority: 12.10.2012 US 201213650933; 12.10.2012 US 201213651038; 12.10.2012 US 201213650774
(43) Date of publication of application: 19.08.2015
(73) Proprietor: L'Oréal, 75008 Paris (FR)
(72) Inventor: LAUTEN, Elizabeth, Hunter, New York, NY 10012 (US); SIMONNET, Jean-Thierry, Mamaroneck, NY 10543 (US); PAN, Zhi, Fort Lee, NJ 07024 (US); BALOOCH, Guive, New York, NY 10014 (US)
(74) Representative: Nony
(86) International application number: PCT/US2013/064466
(87) International publication number: WO 2014/059228

(56) References cited:
- WO-A1-00/78141
- WO-A1-2012/069362
- CN-A- 101 238 886
- JP-A- S6 150 918
- US-A- 4 488 989
- US-A1- 2003 104 080
- US-A1- 2005 276 762
- US-A1- 2006 147 508
- US-B1- 6 221 823
- US-B1- 6 331 520
- DATABASE GNPD [Online] MINTEL; September 2011 (2011-09), "Tro Action B.B Cream", XP002753598, Database accession no. 1632543
- SCHREIER<A> S ET AL: "Surface active drugs: self-association and interaction with membranes and surfactants. Physicochemical and biological aspects", BIOCHIMICA ET BIOPHYSICA ACTA (BBA) - BIOMEMBRANES, ELSEVIER, AMSTERDAM, NL, vol. 1508, no. 1-2, 23 November 2000 (2000-11-23), pages 210-234, XP004273346, ISSN: 0005-2736, DOI: 10.1016/S0304-4157(00)00012-5
- DATABASE GNPD [Online] MINTEL; April 2012 (2012-04), "Eye Serum", Database accession no. 1769125
- DATABASE GNPD [Online] MINTEL; June 2010 (2010-06), "Cellular Energy Day Cream", Database accession no. 1354750
- DATABASE GNPD [Online] MINTEL; September 2007 (2007-09), "Pro-maximiser Anti-Wrinkle Enhancing Care", Database accession no. 822344

## Description

### BACKGROUND OF THE INVENTION

The present invention relates to aqueous cosmetic compositions comprising at least one hydrotrope and at least one active compound. The active compounds that are useful in the compositions of the present invention include phenolic compounds (e.g., polyphenols), adenosine and adenosine analogues, and other compounds that are useful to help curb the effect of aging and skin damage.

### Active Compounds That Are Phenolic Compounds

The formation of free radicals is a widely accepted pivotal mechanism leading to skin aging. Free radicals are highly reactive molecules with unpaired electrons that can directly damage various cellular membranes, lipids, proteins, RNA and DNA. The damaging effects of these reactive oxygen species are induced internally during normal metabolism and externally through various oxidative stresses. UV exposure and environmental pollution can accelerate skin aging by producing free radicals in skin. Antioxidants protect cells from the damage of oxidative stress by scavenging free radicals and inhibiting following oxidation reactions. The topical application of antioxidants is broadly used in skin care products to prevent skin aging.

Phenol/polyphenols, the most abundant antioxidants in diet, are well known as very effective anti-oxidants. They have been widely studied in the prevention of degenerative diseases, particularly cardiovascular diseases and cancers. Many phenol/polyphenols have been formulated in nutrition supplement and consumer products. However, the solubility of most phenol/polyphenols is very limited, especially in water, which diminishes their applications and biological potential in cosmetics. Thus, there is a need for methods of increasing the water solubility of phenol/polyphenols.

Applications and biological potential of many phenol/polyphenols in cosmetics are limited due to their poor solubility. Various delivery systems, such as gel carriers (US application publication 20020086042), or nano crystals (US application publication 2010/0047297), or chemical modification of the polyphenols (US application publications 20090233876, 20080095866, and 20080176956) have been used to obtain better solubility of phenol/polyphenols. However, these approaches have drawbacks. Some are tied to specific delivery systems. Modification of phenol/polyphenols increases costs, the improvement of solubility is still limited, and modifications can reduce the activity of the phenol/polyphenols.

Other solutions to the problem of poor solubility include the use of solubilizers such as strong organic solvents (U.S. Patent 5,532,012) and diterpene glycosides (US application publication 2011/0033525). Nevertheless, these solutions do not have good safety, and are not necessarily compatible with cosmetic formulations. Moreover, most of the time, when water is added to such compositions, the solubility of the phenol/polyphenols decreases dramatically.

Thus, there remains a need for methods for improving the water solubility of phenolic compounds, including polyphenols, for cosmetic and other uses.

### Active Compounds That Are Adenosine or Adenosine Analogues

Adenosine is an endogenous purine nucleoside that modulates many physiological processes. It is known that in regard to stress or injury, adenosine primarily provides cytoprotection preventing tissue damage in disorders like hypoxia, ischemia and seizure (Sebastião AM, Ribeiro JA (2009), "Adenosine receptors and the central nervous system", Handb Exp Pharmacol. 193:471-534) Adenosine is also believed to be an anti-inflammatory agent at one of its cellular signaling receptors, A2A. (Trevethick MA, Mantell SJ, Stuart EF, Barnard A, Wright KN, Yeadon M (October 2008), "Treating lung inflammation with agonists of the adenosine A2A receptor: promises, problems and potential solutions". Br. J. Pharmacol. 155 (4): 463-74)) Topical treatment of adenosine on wound sites can drastically increase tissue repair and reconstruction. (Hasko G, Linden J, Cronstein B, Pacher P (September 2008). "Adenosine receptors: therapeutic aspects for inflammatory and immune diseases". Nat Rev Drug Discov 7 (9): 759-70; Nakav S, Chaimovitz C, Sufaro Y (2008). Bozza, Patricia, ed. "Anti-Inflammatory Preconditioning by Agonists of Adenosine A1 Receptor". PLoS ONE 3 (5): e2107).

In the cosmetic domain, adenosine and its analogues are important active compounds for skin anti-aging due to its function on increasing DNA/protein synthesis in dermal cells. It has been broadly applied in many skin care products to improve the visual appearance of skin, such as soften fine lines and reduce wrinkles of skin and relax the muscles involved in facial movement and expression.

However, the solubility of adenosine is very limited, especially in water, which suppresses its biological potential in cosmetics. Thus, there remains a need for methods for improving the water solubility of adenosine for cosmetic use.

Published U.S. application 20040146474, L'Oreal, discloses methods for softening lines and relaxing the skin with adenosine and adenosine analogues. U.S. patents 6423327 and 6645513, University of Massachusetts, disclose treatment of skin with adenosine or an adenosine analogue. Published U.S. application 20070232561, King Pharmaceuticals Inc., discloses pharmaceutical compositions for promoting wound healing. The pharmaceutical compositions contain high concentration (10 to 70%w/w) of glycols and a thickening agent, and achieved a final concentration of 0.00001 to 0.10% w/w for adenosine analogues.

Published U.S. application 20080219927, A.B. Thakur et al., discloses adenosine derivative formulations for medical imaging. The formulations contain a solvent made up of water and hydroxypropyl-β-cyclodextrin to form a stable composition of adenosine analogues or derivatives that can be used for myocardial perfusion imaging. Published U.S. application 20110152214, Trustees of Tufts College, discloses a silk polymer-based adenosine release system with therapeutic potential for treatment of epilepsy.

### Other Active Compounds

The cosmetic market has begun to include many active ingredients in formulations to help curb the effect of aging and skin damage. Unfortunately, the efficacy of some of these molecules is reduced due to the natural barrier properties of the skin membrane. In particular the outer most stratum corneum layer shows poor skin permeability of compounds that are hydrophilic, very lipophilic, of high molecular weight or charged.

For example, transdermal penetration of active molecules is especially relevant to products designed to protect skin from photoaging. In this case long UVA rays penetrate deep into the epidermis and produce free radicals which can cause long term health effects. Antioxidants are able protect the cells from this damage by scavenging free radicals and inhibiting oxidation reactions. However, research has shown that in order for many of active molecules, such as antioxidants to be effective they must also reside in the epidermis.

The most common technique to increase transdermal delivery is to use penetration enhancers. While many chemical penetration enhancers, such as solvents, work well to disrupt the lamellar lipid structure of the skin, many of them are toxic, irritating, allergenic, or not suited to cosmetic formulations which cover large areas (unlike typical pharmaceutical transdermal patches).

Other techniques to increase transdermal delivery of active compounds rely on delivery systems such as liposomes in combination with solvents. U.S. 2006/0110439 discloses a delivery system containing liposomes and solvents to increase penetration of an active compound. U.S. patent 6,355,657 discloses a system for percutaneous delivery of the opioid loperamide that combines an organic solvent and fatty acid/fatty alcohol penetration enhancers, such as oleic acid, olleyl alcohol, ethoxydiglycol, laurocapram, alkanecarboxylic acid, dimethyl sulfoxide, polar lipids or n-methyl-2pyrrolidone.

While many cosmetic applications require the addition of penetration enhancers for efficacious actives, the enhancers must overcome the added challenges and needs of being safe, reversible and able to work in a cosmetic form or dose. In addition these enhancers must not interfere with the active molecule in such a way that compromises the molecule's activity. Thus, there is a need for compositions that increase the transdermal bioavailability of active compounds.

### BRIEF SUMMARY OF THE INVENTION

The present invention relates to aqueous cosmetic compositions comprising at least one hydrotrope and at least one active compound. The active compounds that are useful in the compositions of the present invention include phenolic compounds (e.g., polyphenols), adenosine and adenosine analogues and other compounds that are useful to help curb the effect of aging and skin damage.

### Active Compounds That Are Phenolic Compounds

The present text discloses aqueous compositions comprising (a) at least one phenolic compound and (b) at least one hydrotrope in an amount effective to solubilize said at least one phenolic compound in water. The hydrotrope can be a cosmetically acceptable hydrotrope, such as nicotinamide, caffeine, sodium PCA, sodium salicylate, urea, or hydroxyethyl urea. The phenolic compound can be any type of phenol or polyphenol.

According to a first aspect, the present invention provides an aqueous cosmetic composition comprising (a) at least one active compound being at least one polyphenol comprising flavones or adenosine ; (b) at least one hydrotrope being nicotinamide and caffeine; and (c) water, wherein said hydrotrope is present in an amount from 0.1% to 20% based on the total weight of the composition to solubilize said at least one active compound in said water.

The text also discloses a method for preparing an aqueous composition comprising including in said composition at least one phenolic compound and at least one hydrotrope in an amount sufficient to solubilize said phenolic compound in water.

According to a second aspect, the present invention provides a method for preparing an aqueous cosmetic composition comprising including in said composition at least one active compound being at least one polyphenol comprising flavones or adenosine ; at least one hydrotrope being nicotinamide and caffeine; and (c) water, wherein said hydrotrope is present in an amount from 0.1% to 20% based on the total weight of the composition to solubilize said active compound in said water.

The text further discloses a method comprising applying an aqueous composition to skin, the aqueous composition comprising (a) at least one phenolic compound and (b) at least one hydrotrope in an amount effective to solubilize the at least one phenolic compound in the water phase.

According to another aspect, the present invention provides a method comprising applying an aqueous cosmetic composition to skin, said aqueous composition comprising (a) at least one active compound being at least one polyphenol comprising flavones or adenosine ; (b) at least one hydrotrope being nicotinamide and caffeine; and (c) water, wherein said hydrotrope is present in an amount from 0.1% to 20% based on the total weight of the composition to solubilize said at least one active compound in said water.

### Active Compounds That Are Adenosine or Adenosine Analogues

One aspect of the present invention provides aqueous compositions comprising (a) at least one compound selected from the group consisting of adenosine and adenosine analogues, and (b) at least one hydrotrope in an amount effective to solubilize the at least one compound of (a) in water. The compound of component (a) can be adenosine, or an adenosine analogue, or any combination of adenosine analogues or adenosine and adenosine analogue(s). The hydrotrope can be a cosmetically acceptable hydrotrope, such as nicotinamide, caffeine, sodium PCA, sodium salicylate, urea, or hydroxyethyl urea.

Another aspect of the invention provides a method for preparing an aqueous composition comprising including in the composition (a) at least one compound selected from the group consisting of adenosine and adenosine analogues, and (b) at least one hydrotrope in an amount effective to solubilize the at least one compound of (a) in water.

A further aspect of the invention provides a method comprising applying an aqueous composition to skin, the aqueous composition comprising (a) at least one compound selected from the group consisting of adenosine and adenosine analogues, and (b) at least one hydrotrope in an amount effective to solubilize said at least one compound (a) in the water phase.

### Other Active Compounds

The present text discloses an aqueous composition comprising a) at least one hydrotrope and b) at least one active compound, wherein the at least one hydrotrope is present in an amount effective to increase transdermal penetration of the active compound. Preferably, the hydrotrope is a cosmetically acceptable hydrotrope, such as nicotinamide, caffeine, sodium PCA, or sodium salicylate. The active compound can be poorly water soluble, requiring the need to be solubilized by the hydrotrope itself (or a combination of hydrotropes), or a hydrophilic molecule that is readily dissolved. Thus, the aqueous compositions of the present invention are effective to increase the bioavailability of the at least one active compound or molecule for topical applications.

The present text further discloses a method for increasing the bioavailability of an active molecule (e.g., in topical applications) comprising applying an aqueous composition to skin, the composition comprising a) at least one hydrotrope and b) at least one active compound, wherein the at least one hydrotrope is present in an amount effective to increase transdermal penetration of the active compound.

Another aspect of the invention provides a method for increasing the bioavailability of an active molecule comprising applying an aqueous composition to skin, said composition comprising a) at least one active compound being at least one polyphenol comprising flavones or adenosine ; b) at least one hydrotrope being nicotinamide and caffeine; and c) water, wherein said hydrotrope is present in an amount from 0.1% to 20% based on the total weight of the composition to increase transdermal penetration of the active compound.

A further aspect of the text discloses a method of preparing an aqueous composition comprising including in the composition at least one hydrotrope and at least one active compound or molecule, wherein the at least one hydrotrope is present in an amount effective to increase transdermal penetration of the active compound.

These and other aspects of the invention are shown in the appended claims, and described in greater detail in the detailed description of the invention.

### Brief Description of the Drawings

FIG. 1 shows a graph of baicalin solubility as a function of nicotinamide concentration.
FIG. 2 shows a graph of baicalin solubility as a function of caffeine concentration.
FIG. 3 shows a diagrammatic representation of the model lipid bilayer assay.
FIG. 4A and FIG. 4B show a table of glucose release from lipids in the presence of hydrotropes, and graph of glucose release after the presence of hydrotropes.
FIG. 5A and FIG. 5B show graphs of the depth of penetration of vitamin C and baicalin in human ex-vivo skin.

### DETAILED DESCRIPTION OF THE INVENTION

The present invention relates to aqueous cosmetic compositions comprising at least one hydrotrope and at least one active compound. The active compounds that are useful in the compositions of the present invention include phenolic compounds (e.g., polyphenols), adenosine and adenosine analogues and other compounds that are useful to help curb the effect of aging and skin damage.

### Hydrotropes

Hydrotropes (or hydrotropic agents) are a diverse class of water-soluble compounds that are characterized by an amphiphilic molecular structure and ability to dramatically increase the solubility of poorly soluble organic molecules in water.

Most hydrotropes have aromatic structure with an ionic moiety, while some of them are linear alkyl chains, as listed in the table below. Although hydrotropes noticeably resemble surfactants and have the ability to reduce surface tension, their small hydrophobic units and relatively shorter alkyl chain distinguish them as a separate class of amphiphiles. Consequently their hydrophobicity is not sufficient enough to create well organized self-associated structures, such as micelles, even with a high concentration.

Common hydrotropic molecules include: sodium 1,3-benzenedisulfonate, sodium benzoate, sodium 4-pyridinecarboxylate, sodium salicylate, sodium benzene sulfonate, caffeine, sodium p-toluene sulfonate, sodium butyl monoglycolsulfate, 4-aminobenzoic acid HCl, sodium cumene sulfonate, *N,N*-diethylnicotinamide, *N*-picolylnicotinamide, N-allylnicotinamide, 2-methacryloyloxyethyl phosphorylcholine, resorcinol, butylurea, pyrogallol, N-picolylacetamide 3.5, procaine HCl, proline HCl, nicotinamide, pyridine, 3-picolylamine, sodium ibuprofen, sodium xylenesulfonate, ethyl carbamate, pyridoxal hydrochloride, sodium benzoate, 2-pyrrolidone, ethylurea, N,N-dimethylacetamide, N-methylacetamide, and isoniazid. Hydrotropes can be found in Lee J. et al., "Hydrotropic Solubilization of Paclitaxel: Analysis of Chemical Structures for Hydrotropic Property", Pharmaceutical Research, Vol. 20, No. 7, 2003; Lee S. et al., "Hydrotropic Polymers: Synthesis and Characterization of Polymers Containing Picolylnicotinamide Moieties", Macromolecules, 36, 2248-2255, 2003; and Hodgon T.K., Kaler E.W., "Hydrotropic Solutions", Current Opinion in Colloid and Interface Science, 12, 121-128, 2007.

Cosmetically acceptable hydrotropes refers to hydrotropes that can be used in cosmetic compositions. While hydrotropes represent a broad class of molecules used in various fields, cosmetic applications will be limited due to safety and tolerance restrictions. Suitable hydrotropes for use in cosmetics include, but are not limited to, the hydrotropes listed below:

| **Name of hydrotropes** | **Structure** |
|---|---|
| Nicotinamide (Vit B3) | 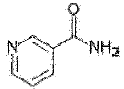 |
| Caffeine | 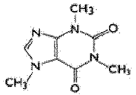 |
| Sodium PCA | 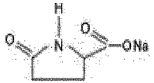 |
| Sodium Salicylate | 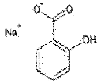 |
| Urea | 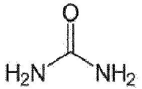 |
| Hydroxyethyl urea | 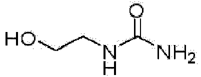 |

The suitability of a hydrotrope for use in cosmetic compositions can be determined using tests known in the art for determining effects of compounds on skin, and toxicity to humans. In certain cases, it may be beneficial to also determine the suitability or desirability of a hydrotrope(s) for use in a cosmetic composition by using tests that measure or estimate the bioavailability of the active compound(s) in the composition when applied to skin in the presence of the hydrotrope(s).

Preferred hydrotropes in cosmetics are listed as below:

| **Name of hydrotropes** | **Structure** |
|---|---|
| Nicotinamide (Vit B3) | 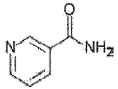 |
| Caffeine | 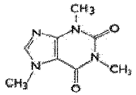 |
| Sodium PCA | 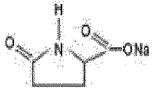 |
| Sodium Salicylate | 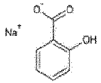 |

### Active Compounds That Are Phenolic Compounds

One aspect of the present text discloses aqueous compositions comprising at least one phenolic compound and at least one hydrotrope for cosmetic and other uses. The hydrotrope, such as a cosmetically acceptable hydrotrope, improves the water solubility of the phenolic compound. The hydrotropes can be used to formulate phenolic compounds, especially polyphenols, in all cosmetic formulas that contain water, for topical application or injection, and food applications, such as beverages.

Most phenolic compounds, including polyphenols, have very limited solubility (<0.1%) in water depending on their various structures. Applicants have discovered that hydrotropes can dramatically increase the solubility of these poorly water soluble phenolic compounds in water by orders of magnitude. The aqueous compositions thus contain phenolic compounds in greater percentage amounts than aqueous compositions in which the hydrotrope is not present. Applicants have also found that combinations of hydrotropes, such as the combination of caffeine and nicotinamide, is more efficient that either one alone for increasing the water solubility of phenolic compounds.

At least one hydrotrope refers to one or a combination of two or more hydrotropes. One or a combination of two or more hydrotropes can be used to improve the solubility of phenolic compounds in water.

The at least one hydrotrope is present in the aqueous composition in amounts effective to increase the solubility of the phenolic compound in water. The amount of hydrotrope will vary depending on the hydrotrope and the type and amount of phenolic compound. The amount of hydrotrope present in the aqueous compositions can range from about 0.1% to about 20%; about 0.1% to about 10%; or about 1% to about 50%, based on the total weight of the composition.

Increasing the water solubility of the phenolic compound(s) refers to increasing the solubility of the phenolic compound(s) in water in comparison with solubility of the phenolic compound(s) in water in the absence of the hydrotrope or hydrotropes.

An advantage of using hydrotropes is, once a stable solution is obtained, further dilution doesn't influence the stability of the solution. This is very different from organic solvents that are commonly used to increase the water solubility of phenolic compounds, such as polyphenols. Typically, an aqueous dilution of organic solvents with pre-dissolved phenolic compound(s), such as a polyphenol, results in crystallization or precipitation.

Phenolic compounds are a structural class of natural, synthetic, and semisynthetic organic compounds that have one or more phenolic constituents. Phenolic compounds containing multiple phenol groups (or phenolic constituents) are known as polyphenols. Polyphenols themselves are a structural class of natural, synthetic, and semisynthetic organic compounds. Polyphenols are normally available in plants and are very helpful to protect plants and also animals from usual health disorders and also the impacts of aging. Polyphenols function as potent free radical scavengers by donating their alcoholic hydrogen or one of their delocalized electrons. The two classes of polyphenols are flavonoids and non-flavonoids.

Flavonoids are a specific group of polyphenols, and are the most plentiful group of polyphenol compounds, making up about two-thirds of the total phenols in consumed feed. Flavonoids are further categorized, according to chemical structure, into chalcones, flavones, flavanones, flavanols, flavonols, dihydroflavonols, isoflavonoids, neoflavonoids, catechins, anthocyanidins, and tannins. Over 4,000 flavonoids have been identified, many of which occur in fruits, vegetables and beverages (tea, coffee, beer, wine and fruit drinks). The flavonoids have been reported to have antiviral, anti-allergic, antiplatelet, anti-inflammatory, antitumor and antioxidant activities. Flavonoids protect lipids and vital cell components from damaging oxidative stress by efficiently scavenging free radicals.

Non-flavonoid polyphenols include lignans, aurones, stilbenoids, curcuminoids and other phenylpropanoids. Many of them are also well-known antioxidants like resveratrol, ferulic acid, curcumin, and pinoresinol.

Other phenolic compounds, in addition to polyphenols, include alkylphenols, betacyanins, capsacinoids, hydroxybenzoketones, methoxyphenols, naphthoquinones, and phenolic terpenes. Some popular examples are ferulic acid, hydroxytyrosol, cinnamic acid, caffeic acid, and *p*-coumaric acid.

The at least one phenolic compound is solubilized in the aqueous compositions, and the amount of phenolic compound will depend on the specific phenolic compound and the type and amount of hydrotrope present in the aqueous compositions. The amount of phenolic compound present in the aqueous compositions can range from about 0.01% to about 20%; about 0.1% to about 20%; or about 0.1% to about 10%, based on the total weight of the composition.

The composition comprises from about 1 to 99.9% by weight of water, with respect to the total weight of the composition. The amount of water in the composition can range from about 1 to 99.5%; about 1 to 60%; or about 1 to 50%, based on the total weight of the composition.

The pH of the aqueous compositions is not limited but is generally between 2 and 12, or between 3 and 9. The pH can be adjusted to the desired value by addition of a base (organic or inorganic) to the composition, for example ammonia or a primary, secondary or tertiary (poly)amine, such as monoethanolamine, diethanolamine, triethanolamine, isopropanolamine or 1,3-propanediamine, or alternatively by addition of an inorganic or organic acid, advantageously a carboxylic acid, such as, for example, citric acid.

Another aspect of the text discloses a method for preparing the aqueous compositions comprising including in the composition at least one phenolic compound and at least one hydrotrope in an amount sufficient to solubilize the phenolic compound, such as a polyphenol, in water. A hydrotrope solution is prepared by completely dissolving one or more hydrotropic agents into water. At least one phenolic compound is then added in and mixed using a stirring bar or any other mixer. Solubilization of the phenolic compound occurs within minutes and mixing is continued until the maximum concentration is achieved, which was defined as the solubility of the phenolic compound(s) under that condition. A clear stable solution with a concentration that does not exceed the solubility would be ready after more than one hour of mixing. No heat is necessary by following this procedure to dissolve phenolic compounds. Everything is prepared at room temperature to keep the stability of the phenolic compound(s). This is extremely useful to protect the activity of certain compounds and also makes the process much easier.

### Active Compounds That Are Adenosine or Adenosine Analogues

One aspect of the present text discloses aqueous compositions comprising (a) at least one compound selected from the group consisting of adenosine and adenosine analogues, and (b) at least one hydrotrope in an amount effective to solubilize said at least one compound (a) in water, for cosmetic uses. The hydrotrope, such as a cosmetically acceptable hydrotrope, improves the water solubility of the adenosine and/or adenosine analogue(s). The hydrotropes can be used to formulate the adenosine and/or adenosine analogue(s) in all cosmetic formulas that contain water, for topical application, injection or oral administration.

Cosmetic uses of the compositions containing adenosine and/or adenosine analogue(s) include antiaging products, skin care products, and products to improve the visual appearance of skin, such as soften fine lines, reduce skin wrinkles, and relax the muscles of the face.

Adenosine and adenosine analogues are poorly water soluble. Applicants have discovered that hydrotropes can dramatically increase the solubility of these poorly water soluble compounds in water by orders of magnitude. The aqueous compositions contain adenosine and/or adenosine analogue(s) in greater percentage amounts than aqueous compositions in which the hydrotrope is not present. Applicants have also found that a combination of hydrotropes, such as the combination of caffeine and nicotinamide, is more efficient than either one alone for increasing the water solubility of adenosine.

At least one hydrotrope refers to one or a combination of two or more hydrotropes. One or a combination of two or more hydrotropes can be used to improve the solubility of adenosine and/or adenosine analogue(s) in water.

The at least one hydrotrope is present in the aqueous composition in amounts effective to increase the solubility of adenosine and/or adenosine analogue(s) in water. The amount of hydrotrope will vary depending on the hydrotrope and the type and amount of adenosine and/or adenosine analogue(s). The amount of hydrotrope present in the aqueous compositions can range from about 0.1 to about 20%; about 0.1 to about 10%; or about 1% to about 50%, based on the total weight of the composition.

Increasing the water solubility of adenosine and/or adenosine analogue(s) refers to increasing the solubility of adenosine and/or adenosine analogue(s) in water in comparison with solubility of the adenosine and/or adenosine analogue(s) in water in the absence of the hydrotrope or hydrotropes.

An advantage of using hydrotropes is that, once a stable solution is obtained, further dilution won't influence the stability. This is very different from organic solvents like glycols that are commonly used to increase the water solubility of adenosine. Typically, an aqueous dilution of organic solvents with pre-dissolved adenosine results in crystallization or precipitation.

The at least one compound selected from the group consisting of adenosine and adenosine analogues can be adenosine, or an adenosine analogue, or any combination of adenosine analogues or adenosine and adenosine analogue(s). Suitable adenosine analogues include agonists of adenosine receptors and compounds increasing intra- or extra-cellular adenosine levels.

Examples of adenosine analogues include: 2'-deoxyadenosine; 2',3'-isopropoylidene adenosine; toyocamycin; 1-methyladenosine, N-6-methyladenosine; adenosine N-oxide; 6-methylmercaptopurine riboside; 6-chloropurine riboside; 5'-adenosine monophosphate; 5'-adenosine diphosphate and 5'-adenosine triphosphate.

Other adenosine analogues include agonists of adenosine receptors, including phenylisopropyl adenosine (PIA), 1-methylisoguanosine, N⁶-cyclohexyl adenosine (CHA), N⁶-cyclopentyl adenosine (CPA), 2-chloro-N-6-cyclopentyladenosine, 2-chloroadenosine, N⁶-phenyladenosine, 2-phenylaminoadenosine, MECA, N⁶-phenethyladenosine, 2-p-(2-carboxyethyl)-phenethyl-amino-5'-N-ethylcarboxamido-adenosine (CGS-21680), N-ethylcarboxamido-adenosine (NECA), 5'-(N-cyclopropyl)-carboxamidoadenosine, DPMA (PD 129,944) and metrifudil.

Other adenosine analogues include compounds which increase the intracellular concentration of adenosine such as erythro-9-(2-hydroxy-3-n-onyl) adenine (EHNA) and iodotubercidin.

Other adenosine analogues also include salts and esters of adenosine.

Adenosine and/or adenosine analogue(s) is or are solubilized in the aqueous compositions, and the amount of adenosine and/or adenosine analogue(s) will depend on the type and amount of the hydrotrope(s) present in the aqueous compositions, as well as the specific adenosine analogue. The amount of adenosine and/or adenosine analogue(s) present in the aqueous compositions can range from about 0.01% to about 20%; about 0.1% to about 10%; or about 0.1 to about 5% based on the total weight of the composition.

The composition comprises from about 1 to 99.9% by weight of water, with respect to the total weight of the composition. The amount of water in the composition can range from about 1 to 99.5%; about 1 to 60%; or about 1 to 50%, based on the total weight of the composition.

The pH of the aqueous compositions is not limited but is generally between 2 and 12, or between 3 and 9. The pH can be adjusted to the desired value by addition of a base (organic or inorganic) to the composition, for example ammonia or a primary, secondary or tertiary (poly)amine, such as monoethanolamine, diethanolamine, triethanolamine, isopropanolamine or 1,3-propanediamine, or alternatively by addition of an inorganic or organic acid, advantageously a carboxylic acid, such as, for example, citric acid.

Another aspect of the text discloses a method for preparing the aqueous compositions comprising including in the composition at least one compound selected from the group consisting of adenosine and adenosine analogue(s) and at least one hydrotrope in an amount sufficient to solubilize the adenosine and/or adenosine analogue(s) in water. A hydrotrope solution is prepared by completely dissolving one or more hydrotropic agents into water. Adenosine and/or adenosine analogue(s) is or are then added in and mixed using a stirring bar or any other mixer. Solubilization of adenosine and/or adenosine analogue(s) occurs within minutes, and mixing is continued until the maximum concentration achieved, which was defined as the solubility of the compound(s) under that condition. A clear stable solution with a concentration that does not exceed the solubility would be ready after more than one hour of mixing. No heat is necessary by following this procedure to dissolve adenosine and/or adenosine analogue(s). Everything is prepared at room temperature. This is extremely useful to protect the activity of certain compounds and also makes the process much easier.

### Other Active Compounds

One aspect of the present text discloses aqueous compositions comprising a) at least one hydrotrope and b) at least one active compound, wherein the at least one hydrotrope is present in an amount effective to increase transdermal penetration of the active molecule. The active compounds in this aspect of the present invention include many (if not all) of the phenolic compounds, adenosine and adenosine analogues discussed earlier, as well as other compounds or molecules.

Applicants have discovered that hydrotropes in cosmetic formulations enhance bioavailability of active molecules. The function of increased bioavailability is due to the ability of the hydrotrope to increase depth of penetration through the skin. Hydrotropes can be used in all cosmetic formulas which contain more than 5% water, for both topical application and injection.

Hydrotropes suitable for use in cosmetic compositions safely and reversibly disrupt the lamellar lipid crystalline layer of the stratum corneum in order to effectively deliver and increase bioavailability of active compounds in cosmetic formulations. When combined with poorly soluble and poorly penetrating active compounds such as polyphenols, adenosine, sugars, and hydrophilic molecules, hydrotropes increase penetration of the active compound through the skin.

As used herein, improving bioavailability of an active compound or molecule refers to increasing the amount of the active compound in skin, in comparison to a composition which does not contain the hydrotrope or hydrotropes present in the claimed aqueous compositions.

As used herein, increasing dermal penetration of an active compound or molecule refers to increasing the amount or depth of penetration, or both, of an active compound through skin, preferably human skin, in comparison with a composition which does not contain the hydrotrope or hydrotropes present in the claimed aqueous compositions, or does not contain such hydrotropes in amounts effective to increase transdermal penetration.

At least one hydrotrope refers to one or a combination of two or more hydrotropes. One or a combination of two or more hydrotropes can be used to improve the bioavailability of active compounds, or increase transdermal penetration of active compounds.

An advantage of using hydrotropes is, once a stable solution is obtained, further dilution doesn't influence the stability of the solution. This is very different from organic solvents that are commonly used to increase the water solubility of actives. Typically, an aqueous dilution of organic solvents with pre-dissolved actives results in crystallization or precipitation.

Hydrotropes which function to increase the penetration of active molecules in the skin can be selected using models of the skin, such as the model lipid bilayer assay disclosed herein the Examples. An effective hydrotrope solution allows the reversible disruption of lipids, taking into consideration the dose and potential combination of cosmetic hydrotropes.

The amount of hydrotrope present in the aqueous compositions will vary depending on the hydrotrope and the type and amount of active compound, in the range of 0.01% to 20%, with respect to the total weight of the composition.

The inventive aqueous compositions comprise at least one active compound from various classes of compounds, such as compounds that are poorly water soluble (solubility < 0.1%), such as polyphenols.

The amount of active compound in the aqueous composition will depend on the identity of the active compound and its solubility in water, and the type and amount of hydrotrope present in the aqueous composition. Preferably, the amount ranges from 0.01% to 20%, based on the total weight of the composition.

When the at least one active compound comprises at least one polyphenol, the at least one polyphenol is solubilized in the aqueous compositions, and the amount of polyphenol will depend on the specific polyphenol and the type and amount of hydrotrope present in the aqueous compositions. The amount of polyphenol present in the aqueous compositions can range from 0.01% to 20%; about 0.1% to about 10%; or about 0.1% to about 5%, based on the total weight of the composition.

Increasing the water solubility of polyphenols refers to increasing the solubility of the polyphenol in water in comparison with solubility of the polyphenol in water in the absence of the hydrotrope or hydrotropes.

If the active compound is poorly soluble in water, the at least one hydrotrope is present in the aqueous composition in amounts effective to increase the solubility of the active compound, such as a polyphenol, in water. The amount of hydrotrope will vary depending on the hydrotrope and the type and amount of the active compound. The amount of hydrotrope present in the aqueous compositions can range from 0.1% to 60%; about 0.1 to about 50%; or about 1% to 50%, based on the total weight of the composition.

The aqueous compositions can comprise at least one active compound and at least one hydrotrope in an amount effective to increase transdermal penetration of the active compound, with water making up the remainder of the composition. Preferably, the composition comprises from 5% to 99.5% by weight of water, with respect to the total weight of the composition.

The pH of the aqueous compositions is not limited but is generally between 2 and 12 and preferably between 3 and 9. The pH can be adjusted to the desired value by addition of a base (organic or inorganic) to the composition, for example ammonia, sodium hydroxide, potassium hydroxide, or a primary, secondary or tertiary (poly)amine, such as monoethanolamine, diethanolamine, triethanolamine, isopropanolamine or 1,3-propanediamine, or with basic amino acids or poly amino acids like lysine, or arginine, or alternatively by addition of an inorganic or organic acid, preferably a carboxylic acid, such as, for example, citric acid.

Another aspect of the text discloses a method for preparing the aqueous compositions comprising including in the composition at least one active compound and at least one hydrotrope in an amount effective to increase transdermal penetration of the active compound. A hydrotrope solution is prepared by completely dissolving one or more hydrotropic agents into water, in an amount effective to increase transdermal penetration of the active compound. If the active compound is poorly soluble in water, the amount of hydrotrope should also be effective to solubilize the active compound. The active compound, such as a polyphenol, is then added in and mixed using stirring bar or any other mixer. A clear stable solution with a concentration that does not exceed the solubility of the active compound would be ready after more than one hour of mixing. No heat is necessary by following this procedure to dissolve polyphenols. Everything is prepared at room temperature to keep the stability of polyphenols. This is extremely useful to protect the activity of certain compounds and also makes the process much easier.

### Aqueous Cosmetic Compositions

The aqueous compositions can also comprise at least one additive conventionally used in the cosmetics field which does not affect the properties of the compositions according to the invention, such as thickeners, fragrances, pearlescent agents, preservatives, sunscreens, anionic or nonionic or cationic or amphoteric polymers, proteins, protein hydrolysates, fatty acids, such as 18-methyleicosanoic acid, vitamins, panthenol, silicones, vegetable, animal, mineral or synthetic oils, gelling agents, antioxidants, solvents, fragrances, fillers, screening agents, odor absorbers and coloring materials. These additives can be present in the composition according to the invention in proportions which are not limited, but which preferably or advantageously fall in the range from 0 to 50% by weight, 5-40% by weight, or 30-50% by weight with respect to the total weight of the composition.

Generally, any composition of the invention can be ingested, injected or topically applied to the skin (over any cutaneous region of the body) or to the mucous membranes (oral, jugal, gingival, genital, conjunctival, and the like). Preferably, the compositions of the invention are topically applied onto the skin or mucous membranes. Depending on the method of administration under consideration, the composition can be provided in any dosage form normally used.

For topical application to the skin, the composition can have the form in particular of aqueous or oily solutions or of dispersions of the lotion or serum type, of emulsions with a liquid or semi-liquid consistency of the milk type, obtained by dispersion of a fatty phase in an aqueous phase (O/W) or vice versa (W/O), or of suspensions or emulsions with a soft consistency of the aqueous or anhydrous gel or cream type, or else of microcapsules or microparticles, or of vesicular dispersions of ionic and/or nonionic type or of foams. These compositions are prepared according to the usual methods. It is preferred that the composition have between 5 to 99.5% of water phase.

For injection, the composition can be provided in the form of aqueous or oily lotions or in the form of serums. For the eyes, the composition can be provided in the form of drops and, for ingestion, it can be provided in the form of capsules, granules, syrups or tablets.

The amounts of the various constituents of the compositions according to the invention are those conventionally used in the fields under consideration.

In the cosmetics field, these compositions constitute in particular creams for cleaning, protecting, treating or caring for the face, for the hands, for the feet, for the major anatomical folds or for the body (for example, day creams, night creams, anti-aging creams, moisturizing creams, make-up-removing creams, foundation creams or sun creams), liquid foundations, make-up-removing milks, protective or care body milks, sun milks, lotions, gels or foams for caring for the skin, such as cleansing lotions, sun lotions, artificial tanning lotions, bath compositions, deodorizing compositions comprising a bactericidal agent, aftershave gels or lotions, depilatory creams, compositions for countering insect stings or bites, pain-relieving compositions or compositions for treating certain skin diseases, such as eczema, rosacea, psoriasis, lichen and severe pruritus.

### EXAMPLES

### EXAMPLE 1

Baicalin, a component of Chinese medicinal herb Huang-chin, is a flavone, a type of flavonoid. It is a potent antioxidant that demonstrates potent effects against oxidative stress diseases, inflammation, allergy, cancer, bacterial infections, etc. However, its solubility in water is extremely low (<0.01% at its natural pH -4.5), especially at low pH, as shown below, and degradation happens at pH>5.

| PH | 3 | 3.5 | 4 | 4.5 | 5 |
|---|---|---|---|---|---|
| Solubility | 0.0016% | 0.0021% | 0.0040% | 0.0084% | 0.035% |

Although certain organic solvents can increase the solubility of baicalin, such as PEG-4 which can dissolve 3% baicalin, a dilution of these solutions in water is not stable any more. Crystallization or precipitation occurs after mixing the glycol phase and water phase.

The solubility of baicalin can be increased by raising the concentration of hydrotropes. And unlike in organic solvents, such solutions are still stable if diluted in water.

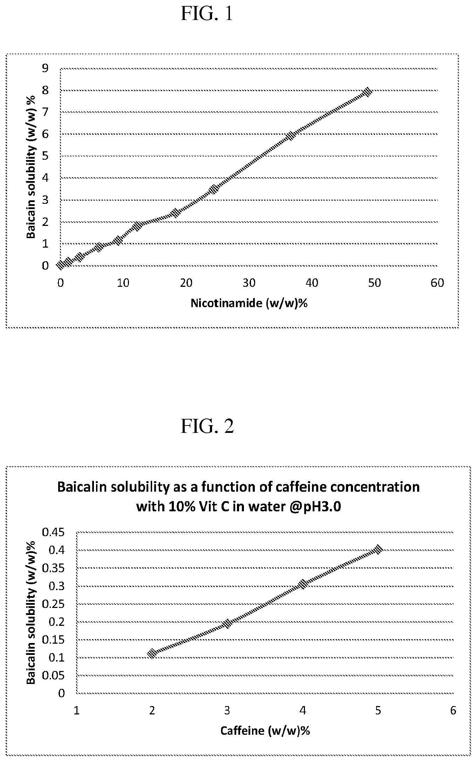

Water solubility of baicalin was increased as a function of nicotinamide concentration as shown in Figure 1.

2% (w/w) caffeine in water improved the water solubility of baicalin from <0.01% to 0.11%; and further improvement was observed as more caffeine was dissolved in water with 10% Vit C, shown in Figure 2.

### EXAMPLE 2 - Combination of nicotinamide and caffeine

The water solubility of caffeine is approximately 2%, which limited its function as a hydrotropic agent. By mixing with nicotinamide, the solubility of caffeine can be increased to 5% or higher. And the combination of caffeine and nicotinamide is more efficient than any one of themselves. The combination of 5% nicotinamide and 5% caffeine in water solubilized approximately 1% baicalin in water, which dramatically increased the water solubility of baicalin by more than 100 times.

After the hydrotrope solution was prepared at certain concentrations by completely dissolving one or more hydrotropic agents into water, phenolic compounds were added in and mixed using stirring bar or any other mixer, solubilization happened in minutes and kept going on till the maximum concentration achieved, which was defined as the solubility of the phenolic compound under that condition. A clear stable solution with a concentration that does not exceed the solubility would be ready after >1 hour mixing. No heat is necessary by following this procedure to dissolve phenolic compounds. Everything is prepared at room temperature to keep the stability of the phenolic compounds. This is extremely useful to protect the activity of certain compounds and also makes the process much easier.

The association of hydrotropes, 5% nicotinamide and 5% caffeine, have been found to be very efficient to increase the water solubility of numerous polyphenols, including flavonoid and non-flavonoid polyphenols, and other phenolic compounds.

The results are listed in the table below:

| Phenolic compound | Type | Solubility in water without hydrotropes % (w/w) | Solubility in water with hydrotropes % (w/w) |
|---|---|---|---|
| Baicalin | flavones | <0.01 | >1 |
| Taxifolin | dihydroflavonols | <0.1 | >1 |
| Neohesperidin | dihydrochalcone | <0.05 | >1 |
| Resveratrol | stilbenoids | <0.005 | >0.75 |
| Ellagic acid | tannins | <0.001 | >0.01 |
| Ferulic acid | Phenolic acid | <0.1 | >2 |

### EXAMPLE 3 - Compatibility of the polyphenol/hydrotrope complex in different systems

### Preparation A: Serum

| Phase | Component | Weight % of total |
|---|---|---|
| A | Propylene glycol | 10 |
| A | Dipropylene glycol | 10 |
| A | Ethanol | 10 |
| B | Water | 59.5 |
| B | Nicotinamide | 5 |
| B | Caffeine | 5 |
| B | Baicalin | 0.5 |

Preparation A was prepared as follows. The glycol phase (Phase A) components were mixed together at room temperature. At the same time, the aqueous phase (Phase B) components were mixed at room temperature until a clear solution was obtained. The glycol phase was then added into the aqueous phase with constant stirring for another one hour, and the desired serum was obtained.

### Preparation B: O/W (oil in water) emulsion (cream)

| Phase | Component | Weight % of total |
|---|---|---|
| A1 | Water | 58.5 |
| A1 | Nicotinamide | 5 |
| A1 | Caffeine | 5 |
| A1 | Baicalin | 0.5 |
| A2 | Glycerin | 10 |
| A2 | Xanthan gum | 0.2 |
| A2 | Preservatives | 1 |
| B | Dicaprylyl carbonate | 3 |
| B | Dimethicone | 3 |
| B | Dicapryl alcohol and ceteareth-20 | 4 |
| B | Glyceryl stearate and PEG-100 stearate | 4.5 |
| C | Dimethicone ammonium | 4 |
| C | Polyacryloyldimethyl taurate | 0.3 |
| D | Nylon-12 | 1 |

Preparation B was prepared as follows. Phase A1 components were mixed at room temperature until clear solution was obtained. In separate containers, Phase A2 was presuspended and then added into Phase A1 with constant stirring and heated to 65 °C. At the same time, Phase B components were mixed and completely dissolved at 65 °C. Then Phase B was added into Phase A and emulsified for 10-15 minutes. Heating was stopped, and mixing was continued when Phase C was added and mixed for another 10 minutes. Phase D was added after the temperature was below 40 °C, and mixed for 10-15 minutes (side sweep) or until powders were fully dispersed, and the desired emulsion was obtained.

### Preparation C: W/Si emulsion (gel)

| Phase | Component | Weight % of total |
|---|---|---|
| A | BIS-PEG/PPG-14/14 DIMETHICONE (and) DIMETHICONE | 4 |
| A | Dimethicone (and) dimethiconol | 1 |
| A | Dimethicone | 10 |
| B1 | Water | 43.95 |
| B1 | Nicotinamide | 5 |
| B1 | Caffeine | 5 |
| B1 | Baicalin | 0.5 |
| B2 | Glycerin | 15 |
| B2 | Propylene glycol | 5 |
| B3 | Water | 5 |
| B3 | Preservatives | 0.25 |
| B3 | Sodium citrate | 0.2 |
| B3 | Sodium chloride | 0.8 |
| C | Ethanol | 3 |
| C | Preservatives | 0.6 |
| D | Silica silylate | 0.7 |

Preparation C was prepared as follows. Phase A components were mixed together at room temperature. Phase B1 and Phase B2 were premixed in separate containers at room temperature until clear solutions were obtained. Phase B3 was mixed while heating it to 75-80 °C until it was clear. Phase B2 and Phase B3 were added into Phase B1 while mixing. Then Phase B was slowly added into Phase A while mixing (as viscosity increased, the mixing speed was appropriately increased). When the addition was finished, mixing was continued for an additional 10 minutes before adding pre-mixed Phase C. Phase D was slowly added while mixing until it was thoroughly dispersed, and the desired emulsion was obtained.

### EXAMPLE 4 - Model lipid bilayer assay

In this model system the lipid bilayers are able entrap a detectable compound and upon lipid disorder (i.e. in the presence of a hydrotrope) the compound can be released from the structure and measured in a chemical assay.

A representative lipid mixture (POLYGLYCERYL-3 CETYL ETHER (47.5%) (and) CHOLESTEROL (47.5% (and) DICETYL PHOSPHATE 5%) was selected for the lipid vesicles and glucose was chosen as the entrapped compound. Glucose release can be measured by glucose-oxidase assay using a Sigma HK assay kit. Higher glucose release in the presence of a hydrotrope suggests that that particular hydrotrope could have a greater effect in penetration improvement.

The experimental setup is as follows: 1% lipids were added to a 0.3M glucose solution and stirred for 6 hours (rpm 1100) at 85 °C. Final lipid solution was dialysized for 24hrs in 0.7% NaCl bath using a 3.5Kd dialysis membrane in order to remove any free glucose in the supernatant. To ensure the remaining glucose is fully entrapped in the lipid vesicle the supernatant is measured by the glucose oxidase kit. As a control the lipid vesicles can be broken by n-octylglucoside and the entrapped glucose can be measured.

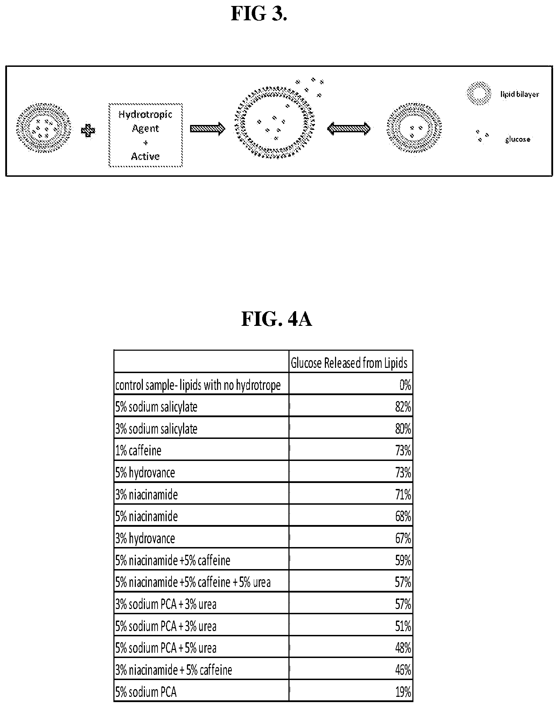

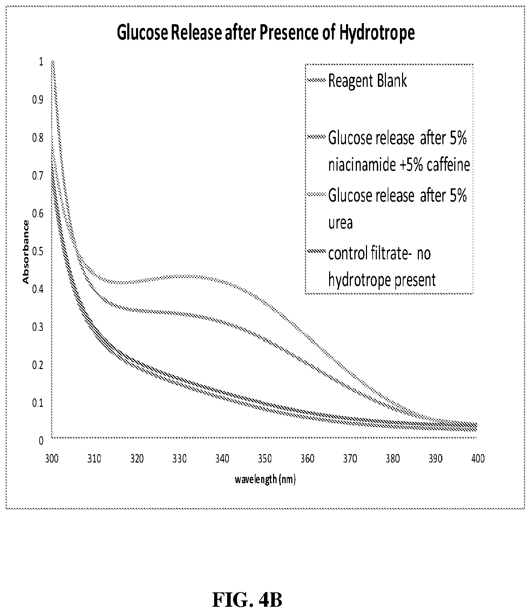

The results shown in Figure 4 (4A and 4B) represent a lipid suspension that was incubated with hydrotropes for 20 hours. The higher release of glucose suggests a more efficient hydrotrope or combination of hydrotropes. The following hydrotropes were tested both for dose response and potential synergy when combined with each other: caffeine, niacinamide, NaPCA, and sodium salicylate.

Hydrotropes or combinations of hydrotropes with >15% glucose release were selected as preferred hydrotropes for formulations.

### EXAMPLE 5 - Ex-Vivo Testing of hydrotropes as penetration enhancers

To further evaluate the effect of penetration enhancers on human skin we performed CARS (coherent anti-stokes raman spectroscopy) experiments. Using this technique we are able to follow the penetration depth of antioxidants in human ex-vivo skin and compare the variability between a formulation containing no hydrotropes, one hydrotrope, and combination of hydrotropes.

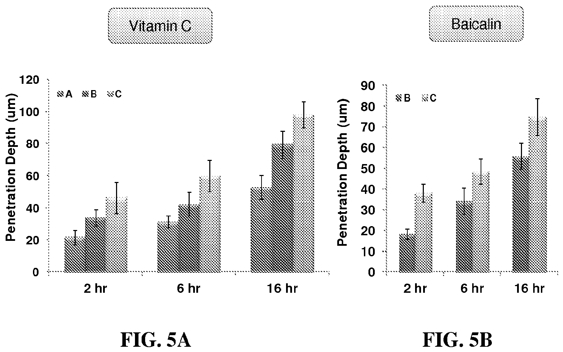

The results in Figure 5 (5A and 5B) show the penetration depth of the antioxidants vitamin C and baicalin (solubilised by niacinamide and caffeine) in ex-vivo human skin after 2, 6, and 16 hours with the following formulas- Formula A (glycol serum with 10% vitamin C), Formula B (glycol serum with 10% vitamin C, 0.5% baicalin, and 5% caffeine), and Formula C (glycol serum with 10% vitamin C, 0.5% baicalin, 5% caffeine and 5% niacinamide). Formula A does not contain baicalin as it requires the addition of some hydrotropes for aqueous solubility. In Figure 5A, Formula A is represented by the left column; Formula B by the middle column; and Formula C by the right column. In Figure 5B, Formula B is represented by the left column; and Formula C by the right column.

The results clearly show that the addition of hydrotropes to formulations increases the penetration depth of the antioxidant molecules. In this case the synergy of niacinamide and caffeine allow for the highest penetration depth of both vitamin C and baicalin.

Of interesting note, baicalin requires the association of hydrotropes in order to achieve efficient levels in aqueous environments while vitamin C is highly hydrophilic and does not. However, the addition of hydrotropes in the final formulation improves the penetration depth of both molecules.

### EXAMPLE 6- Compatible Formula Compositions

### 6.1 Serum

The serum was prepared using the following method:

| Phase | Component (INCI US Name) | Concentration (wt% of total) |
|---|---|---|
| A | DIPROPYLENE GLYCOL | 10 |
| A | PEG-4 | 10 |
| A | BAICALIN | 0.4 |
| B | ALCOHOL DENATURED | 10 |
| C | WATER | 49.5 |
| C | ASCORBIC ACID | 10 |
| C | CAFFEINE | 5 |
| C | NIACINAMIDE | 5 |
| C | BAICALIN | 0.1 |
| PH | SODIUM HYDROXIDE | 0 |

The compounds of phase A were combined and heated to -65-75 °C under constant stirring. Phase A was then cooled and phase B was added. Separately, the compounds of phase C were combined and mixed under constant stirring at room temperature. Phase A+B was added to phase C and stirred for one hour to form the serum. The pH of the serum was adjusted to 4.5 with sodium hydroxide .

### 6.2 Oil in Water Emulsion

The oil in water emulsion was prepared using the following method:

| Phase | Component (INCI US Name) | Concentration (wt% of total) |
|---|---|---|
| A | WATER | 46.78 |
| A | NIACINAMIDE | 2.7 |
| A | CAFFEINE | 2.7 |
| A | BAICALIN | 0.27 |
| A | POLYSORBATE 61 | 0.36 |
| A | PHENOXYETHANOL | 0.5 |
| A | SODIUM STEAROYL GLUTAMATE | 0.4 |
| A | DISODIUM EDTA | 0.1 |
| B | OCTOCRYLENE | 2.5 |
| B | ISOPROPYL LAUROYL SARCOSINATE | 5 |
| B | OCTYLDODECANOL (and) OCTYLDODECYL XYLOSIDE | 0.86 |
| B | GLYCERYL STEARATE | 0.29 |
| B | STEARYL ALCOHOL | 0.14 |
| B | CAPRYLYL GLYCOL | 0.3 |
| B | p-ANISIC ACID | 0.1 |
| B | ISOTRIDECYL ISONONANOATE | 32 |
| B | BASF WRP | 5 |

The compounds of phase A were combined and heated to 80-85°C mixing slowly (500-700 rpm) until all dissolved. Separately, the compounds of phase B were combined and heated to ∼80-85°C mixing slowly (500-700 rpm) until all dissolved. Phase B was added to phase A under mixing conditions (Rayneri). The mixing speed was increased and the mixture was emulsified for thirty minutes. Particle size was then checked under a microscope. The resultant oil in water emulsion was cooled to 25°C with slower speed mixing. The emulsion was then run under a high pressure homogenizer.

### EXAMPLE 7

After the hydrotrope solution was prepared at certain concentrations by completely dissolving one or more hydrotropic agents into water, adenosine was added in and mixed using stirring bar or any other mixer, solubilization happened immediately and kept going on until the maximum concentration achieved, which was defined as the solubility of adenosine under that condition. A clear stable solution with a concentration that does not exceed the solubility would be ready after >1 hour mixing. No heat is necessary by following this procedure to dissolve adenosine. Everything is prepared at room temperature.

The water solubility of caffeine is -2%, which limits its function as a hydrotropic agent. By mixing with nicotinamide, the solubility of caffeine can be increased to 5% or even higher. And the combination of caffeine and nicotinamide is more efficient than any one of themselves. Here the combination of 5% nicotinamide and 5% caffeine in water was used to solubilize adenosine, and a stable clear solution with 3% (w/w) adenosine was obtained.

| Name | Type | Solubility in water without hydrotropes %(w/w) | Solubility in water with hydrotropes %(w/w) |
|---|---|---|---|
| Adenosine | nucleoside | <0.5 | >3 |

As shown in above table, the solubility of adenosine in water was dramatically increased. Additionally, this solution of adenosine/hydrotropes in water is stable to be further diluted or concentrated in cosmetic formulas once we keep the ratio between hydrotropes and adenosine. Therefore, this solubility can be the final weight concentration in cosmetic formulae.

### EXAMPLE 8 Preparation A:

| Phase | Component | Weight % of total |
|---|---|---|
| A | BIS-PEG/PPG-14/14 DIMETHICONE (and) DIMETHICONE | 4 |
| A | Dimethicone (and) dimethiconol | 1 |
| A | Dimethicone | 10 |
| B1 | Water | 43.95 |
| B1 | Nicotinamide | 5 |
| B1 | Caffeine | 5 |
| B1 | Adenosine | 3 |
| B2 | Glycerin | 15 |
| B2 | Propylene glycol | 5 |
| B3 | Water | 5 |
| B3 | Preservatives | 0.25 |
| B3 | Sodium citrate | 0.2 |
| B3 | Sodium chloride | 0.8 |
| C | Ethanol | 3 |
| C | Preservatives | 0.6 |
| D | Silica silylate | 0.7 |

Preparation A was prepared as follows. Phase A components were mixed together at room temperature. Phase B1 and Phase B2 were premixed in separate containers at room temperature until clear solutions were obtained. Phase B3 was mixed while heated to 75-80 °C until it was clear. Phase B2 and Phase B3 were added into Phase B1 while mixing. Then Phase B was slowly added into Phase A while mixing (as viscosity increased, the mixing speed was appropriately increased). When the addition was finished, mixing was continued for an additional 10 minutes before adding pre-mixed Phase C. Phase D was slowly added while mixing until it was thoroughly dispersed, and the desired emulsion was obtained.

## Claims

1. An aqueous cosmetic composition comprising (a) at least one active compound being at least one polyphenol comprising flavones, or adenosine; (b) at least one hydrotrope being nicotinamide and caffeine; and (c) water, wherein said hydrotrope is present in an amount from 0.1% to 20% based on the total weight of the composition to solubilize said at least one active compound in said water.

2. The composition of claim 1, wherein said active compound is present in said composition in an amount of between 0.01% to 20% based on the total weight of the composition.

3. A method for preparing an aqueous cosmetic composition comprising including in said composition at least one active compound being at least one polyphenol comprising flavones, or adenosine; at least one hydrotrope being nicotinamide and caffeine; and (c) water, wherein said hydrotrope is present in an amount from 0.1% to 20% based on the total weight of the composition to solubilize said active compound in said water.

4. A method comprising applying an aqueous cosmetic composition to skin, said aqueous composition comprising (a) at least one active compound being at least one polyphenol comprising flavones, or adenosine; (b) at least one hydrotrope being nicotinamide and caffeine; and (c) water, wherein said hydrotrope is present in an amount from 0.1% to 20% based on the total weight of the composition to solubilize said at least one active compound in said water.

5. The method of claim 4, wherein said at least one active compound is at least one polyphenol comprising flavones.

6. A method for increasing the bioavailability of an active molecule comprising applying an aqueous composition to skin, said composition comprising a) at least one active compound being at least one polyphenol comprising flavones, or adenosine; b) at least one hydrotrope being nicotinamide and caffeine; and c) water, wherein said hydrotrope is present in an amount from 0.1% to 20% based on the total weight of the composition to increase transdermal penetration of the active compound.

7. The composition of any one of claims 1 or 2, wherein the at least one active compound is a polyphenol comprising flavones being present in an amount from 0.1 to 10% based on the total weight of the composition.

8. The composition of any one of claims 1 or 2, wherein the at least one active compound is adenosine being present in an amount from 0.1 to 10% based on the total weight of the composition.

## Patentansprüche

1. Wässrige kosmetische Zusammensetzung, umfassend (a) mindestens einen Wirkstoff, bei dem es sich um mindestens ein Polyphenol, umfassend Flavones oder Adenosin handelt; (b) mindestens ein Hydrotropikum, bei dem es sich um Nicotinamid und Koffein handelt; und (c) Wasser, wobei das Hydrotropikum in einer Menge von 0,1 % bis 20 %, bezogen auf das Gesamtgewicht der Zusammensetzung, zur Solubilisierung des mindestens einen Wirkstoffs in dem Wasser vorliegt.

2. Zusammensetzung nach Anspruch 1, wobei der Wirkstoff in der Zusammensetzung in einer Menge zwischen 0,01 % bis 20 %, bezogen auf das Gesamtgewicht der Zusammensetzung, vorliegt.

3. Verfahren zur Herstellung einer wässrigen kosmetischen Zusammensetzung, bei dem man in die Zusammensetzung mindestens einen Wirkstoff, bei dem es sich um mindestens ein Polyphenol umfassend Flavones, oder Adenosin handelt; mindestens ein Hydrotropikum, bei dem es sich um Nicotinamid und Koffein handelt; und (c) Wasser einarbeitet, wobei das Hydrotropikum in einer Menge von 0,1 % bis 20 %, bezogen auf das Gesamtgewicht der Zusammensetzung, zur Solubilisierung des Wirkstoffs in dem Wasser vorliegt.

4. Verfahren, bei dem man eine wässrige kosmetische Zusammensetzung auf Haut aufbringt, wobei die wässrige Zusammensetzung (a) mindestens einen Wirkstoff, bei dem es sich um mindestens ein Polyphenol, umfassend Flavones, oder Adenosin handelt; (b) mindestens ein Hydrotropikum, bei dem es sich um Nicotinamid und Koffein handelt; und (c) Wasser umfasst, wobei das Hydrotropikum in einer Menge von 0,1 % bis 20 %, bezogen auf das Gesamtgewicht der Zusammensetzung, zur Solubilisierung des mindestens einen Wirkstoffs in dem Wasser vorliegt.

5. Verfahren nach Anspruch 4, bei dem es sich bei dem mindestens einen Wirkstoff um mindestens ein Polyphenol umfassend Flavones handelt.

6. Verfahren zur Erhöhung der Bioverfügbarkeit eines Wirkmoleküls, bei dem man eine wässrige Zusammensetzung auf Haut aufbringt, wobei die Zusammensetzung a) mindestens einen Wirkstoff, bei dem es sich um mindestens ein Polyphenol, umfassend Flavones, oder Adenosin handelt; b) mindestens ein Hydrotropikum, bei dem es sich um Nicotinamid und Koffein handelt; und c) Wasser umfasst, wobei das Hydrotropikum in einer Menge von 0,1 % bis 20 %, bezogen auf das Gesamtgewicht der Zusammensetzung, zur Erhöhung der transdermalen Penetration des Wirkstoffs vorliegt.

7. Zusammensetzung nach einem der Ansprüche 1 bis 2, wobei es sich bei dem mindestens einen Wirkstoff um ein Polyphenol umfassend Flavones handelt, das in einer Menge von 0,1 bis 10 %, bezogen auf das Gesamtgewicht der Zusammensetzung, vorliegt.

8. Zusammensetzung nach einem der Ansprüche 1 bis 2, wobei es sich bei dem mindestens einen Wirkstoff um Adenosin handelt, das in einer Menge von 0,1 bis 10 %, bezogen auf das Gesamtgewicht der Zusammensetzung, vorliegt.

## Revendications

1. Composition cosmétique aqueuse comprenant (a) au moins un composé actif qui est au moins un polyphénol comprenant des flavones, ou l'adénosine ; (b) au moins un hydrotrope qui est le nicotinamide et la caféine ; et (c) de l'eau, où ledit hydrotrope est présent selon une quantité allant de 0,1 % à 20 % sur la base du poids total de la composition, afin de solubiliser ledit au moins un composé actif dans ladite eau.

2. Composition selon la revendication 1, dans laquelle ledit composé actif est présent dans ladite composition selon une quantité allant de 0,01 % à 20 %, sur la base du poids total de la composition.

3. Procédé de préparation d'une composition cosmétique aqueuse comprenant l'incorporation dans ladite composition d'au moins un composé actif qui est au moins un polyphénol, comprenant des flavones, ou l'adénosine ; au moins un hydrotrope qui est le nicotinamide et la caféine ; et (c) de l'eau, où ledit hydrotrope est présent selon une quantité allant de 0,1 % à 20 % sur la base du poids total de la composition, afin de solubiliser ledit composé actif dans ladite eau.

4. Procédé comprenant l'application d'une composition cosmétique aqueuse à de la peau, ladite composition aqueuse comprenant (a) au moins un composé actif qui est au moins un polyphénol, comprenant des flavones, ou l'adénosine ; (b) au moins un hydrotrope qui est le nicotinamide et la caféine ; et (c) de l'eau, où ledit hydrotrope est présent selon une quantité allant de 0,1 % à 20 % sur la base du poids total de la composition, afin de solubiliser ledit au moins un composé actif dans ladite eau.

5. Procédé selon la revendication 4, dans lequel ledit au moins un composé actif est au moins un polyphénol.

6. Procédé destiné à améliorer la biodisponibilité d'une molécule active, comprenant l'application d'une composition aqueuse à de la peau, ladite composition comprenant (a) au moins un composé actif qui est au moins un polyphénol, comprenant des flavones, ou l'adénosine ; (b) au moins un hydrotrope qui est le nicotinamide et la caféine ; et (c) de l'eau, où ledit hydrotrope est présent selon une quantité allant de 0,1 % à 20 % sur la base du poids total de la composition, afin d'augmenter la pénétration transdermique du composé actif.

7. Composition selon l'une quelconque des revendications 1 à 2, dans laquelle le au moins un composé actif est un polyphénol comprenant des flavones qui est présent selon une quantité allant de 0,1 à 10 % sur la base du poids total de la composition.

8. Composition selon l'une quelconque des revendications 1 à 2, dans laquelle le au moins un composé actif est l'adénosine qui est présente selon une quantité allant de 0,1 à 10 % sur la base du poids total de la composition.
